# EUROPEAN PATENT APPLICATION

(11) **EP 1 365 241 A2**
(43) Date of publication of application: **26.11.2003**
(21) Application number: 01978029.5
(22) Date of filing: 20.08.2001
(51) Int. Cl.: G01N 33/553

(54) **METHOD AND DEVICE FOR SIMULTANEOUS DETECTION OF MULTIPLE COMPONENTS IN A MIXTURE**

(30) Priority: 21.08.2000 BY 20000790; 21.08.2000 BY 20000791; 13.10.2000 BY 20000935
(71) Applicant: Darashkevitch, Oleg, Minsk, 220088 (BY)
(72) Inventor: DARASHKEVITCH, Oleg N., Minsk, 220088 (BY); BADEIKO, Anatoly G., Minsk, 220040 (BY); SHAKHNENKO, Pavel P., Minsk, 220102 (BY)
(74) Representative: Helino, Timo
(86) International application number: BY0100014
(87) International publication number: WO02021130

(57) **Abstract**

The invention relates to the field of ecology, biotechnology, molecular biology, biological chemistry, immunology, in particular to detection of components with the help of specific interactions between the latter and a microcarrier comprising a receptor immobilized thereon for defining said components.

Increasing the reliability of simultaneous detection of a number of components in a mixture, making the analysis cheaper and less time-consuming, extending the range of markers used as well as providing the possibility of visual registration of the contents of a number of components in a mixture is achieved by a method and a device for simultaneous detection of a number of components in a mixture, said method comprising a step of reacting of the components under analysis with the specific receptors immobilized on the surfaces of microcarriers of various forms. As a result of formation of specific complexes a mobility of microcarriers under the influence of a physical field is changed. Due to the change of mobility of microcarriers under the influence of a physical field a separation into a fraction of non-reacted microcarriers with unchanged mobility, and a fraction or fractions of microcarriers with changed mobility. Since each type of a specific receptor is bound to its marked microcarrier the analysis of just a marker characteristic of microcarriers with changed mobility under the influence of a physical field will reveal the availability of the component which has reacted with this microcarrier in the mixture under analysis. Under a physical field as in the case described a gravitation field, an electric field or a magnetic field as well as combinations of .the above is understood.

A more efficient reacting of the mixture components with a minimum amount of microcarriers , provision of an efficient and operation-saving separation of microcarriers is achieved in a convenient for sterilization separating chamber where the process of retaining the microcarriers on the walls of a separating chamber and removing them into the collector is actually continuous.

## Description

### Field of the Invention

The invention relates to the field of ecology, biotechnology, molecular biology, biological chemistry, immunology, in particular to detection of components with the help of specific interactions between the latter and a microcarrier comprising a receptor immobilized thereon for defining said components.

### Prior Art

Known is a method for simultaneous analysis of a number of analytes in one sample described in International Patent Application N 89 11101 where the monodispersed particles carrying a specific affinity receptor are analyzed for each individual component, and a marked ligand is used for evaluation of an amount of the component bound. Thus each component is analyzed by a pair of particles of different types having identical specificity, but different binding capacity, and the differences in the sizes of particles of different types are defined by the character of light scattering caused by the particles during their passage through a phototocell of a cytoflowmeter.

Identical is a method for simultaneous analysis of a number of analytes in one sample described in Russian Federation patent N2111488.

The disadvantages of these methods are as follows:
a) relatively low sensitivity,
b) limited amount of components analyzed simultaneously.

US patent N 5891738 discloses a biospecific multiparameter method for analysis and a device for its implementation which are the nearest analogues of a method and a device filed. The method is based on the use of different categories of microparticles as a solid phase presenting various components under analysis where each category of microparticles is coated with different primary biospecific receptors. These microparticles are added with one or a number of fluorescent indicators in one or a number of concentrations or the structure of said microparticles contains said indicators. During analysis the use is made of the secondary biospecific reagents marked by a photoluminescent marker. A method is implemented in the following sequence:
1. Mixing microparticles of different types in a suspension and adding a sample to the suspension;
2. Adding a mixture of secondary biospecific reagents marked by a photoluminescent marker to a suspension with the aim to initiate a biospecific reaction between the components under analysis and the marked reagents with the resulting products of the reaction bound with microparticles,
3. A dilution of a suspension for decreasing the concentration of marked reagents which have not bound with particles,
4. Activation of indicators and of a photoluminescent marker,
5. Registration of photon emission spectra of the indicator for microparticle category identification and of photon emission spectra of the marker and emission intensity for detection of the component concentration.
   However, this method has the following disadvantages:
   1. It is impossible to reliably define the lack of an individual component in a mixture which is especially important during the analysis of complex mixtures;
   2. Besides marked particles it is also necessary to have marked biospecific reagents for each component, this making the analysis substantially more expensive;
   3. Due to the necessity to analyze all particles in a suspension the time needed for the analysis becomes substantially longer;
   4. The exclusive use of fluorescent dyes as indicators diminishes the possibilities of registering the differences between microcarriers,

In all the references cited the reacting of the components under analysis with the specific receptors immobilized on the surfaces of microcarriers is carried out. .Also known are methods and devices for such reacting described in European patent EP - 140787 and International Patent Applications WO - 86/06493, 87/01608, 87/05536, 92/04961, 94/15694, 94/18565, 96/26782, 99/59694, 99/59695, 00/01462.

However, to provide complete separation of a desirable component from a mixture using the methods and the devices described relatively large amounts of magnetic particles with the ability to bind with this component are needed. To achieve a dynamic equilibrium between bound and unbound fractions of the component a long-term stirring of the concentrated mixture with the particles is required.

Known are a method and a device disclosed in Russian Federation patents N 2138801 (27.09.99 Bulletin N27) NN 2142631, 2142632 and 2142633 (10.12.99 Bulletin N 34) for separation of magnetic particles from a particle-containing mixture. A device comprises a mixture container and a magnetic means for separation of particles, said means supplied with a protective cover and a magnet which is made movable relative to the protective cover, and said device is additionally supplied with a means for concentration of particles on a container wall thus providing the collection of particles from this area. The above-mentioned patents also describe a method for separating the particles from a mixture in a container, said method comprising the steps of collecting the particles with the help of a magnetic separation means which the particles are retained thereon and subsequent removal of the particles from said magnetic means by removing the effect of a magnetic field where prior to their collecting the particles have been concentrated on a container wall and resulting removal of the particles with the help of said means from this area.

However, a device and a method described presume the use of an excess of specifically bound magnetic particles to achieve a complete sorption of desirable components and do not make it possible to carry out their efficient concentration with a minimum amount of magnetic particles. With regard to high cost of magnetic particles this leads to a considerable increase of the analysis costs.

Known are the chambers for separation of microcarriers described in USSR inventor's certificates NN1057074, 1487994, 1519753, 1669556, and Russian Federation Patents NN2106896 and 2146551 comprising a tank for purified fluid, inlet and outlet fittings, a fitting for exhausting the mixtures, a rotor-rotating magnetic system and sludge-catching elements. A magnetic system described in USSR inventor's certificate N1554940 is designed in the form of a segment covering a rotating pipeline.

However, all these devices are intended mostly for purifying of technical fluids and are unsuitable for isolation of biological substances sensitive to mechanical influences.

Known is a device for continuous magnetic separation described in US Patent N4663029, said device comprised of a nonmagnetic separating chamber with a magnetizing wire located near a narrow lateral wall of the separating chamber. The wire magnetized under the influence of an external magnetic field generates a field magnetic component which is ortogonal to the longitudinal axis of the wire. A magnetic gradient is distributed along the whole space of the separating chamber, and thus it influences the particles passing through the separating chamber with a fluid flow. Depending on the orientation of the magnetic field in the separating chamber diamagnetic and paramagnetic particles of a mixture are attracted to the opposite walls of the separating chamber and collected in separate collectors.

However, this device proved to be inefficient in real operating conditions due to the turbulence of a fluid flow in the separating chamber.

A device disclosed in US patent N5536475 for magnetic separation of cells consists of two reservoirs and a base with a shaker movably fixed thereon. On the shaker there is positioned a container whereto the mixture of cells and superparamagnetic particles from the first reservoir is supplied. Thus in extreme positions of the shaker the particles are either attracted to a wall of the container with the help of a permanent magnet or released to a washing fluid supplied from the second reservoir.

However, the above-described device is unsuitable for continuous magnetic separation of relatively large volumes of fluid mixtures.

A device for continuous magnetic separation of components from the mixtures disclosed in US patent N6036857 comprises at least one separating chamber with a number of channels and a number of permanent magnets. This chamber is used for periodic magnetic retaining of superparamagnetic particles with the components bound to them by an affinity bond. Each cycle of retaining is completed by a spatial moving away of the separating chamber from permanent magnets. Thus the components retained are supplied into the chamber for regeneration of particles and for releasing the mixture components bound to them.

The disadvantage of the above-mentioned device is its cyclical rate of the processes of retaining and releasing the components which in its turn requires rather a sophisticated control of the flows of an original mixture, washing out solutions and separated components.

Known is a device disclosed in Int. Patent Application N00/01462 representing a slowly rotating horizontal chamber consisting of a number of combined magnetic parts. Each part consists of an alternating current coil covering the chamber and a pair of permanent magnets positioned further along a fluid flow and rotating together with the chamber. The rotation of the chamber stimulates a low gravitation state sharply slowing down the process of sedimentation of particles in a separated mixture. This results in a good contact of affinity paramagnetic particles with nonmagnetic mixture components specifically reacting with them. An alternative magnetic field gradient generated by alternating current coils provides forward and rotary fluctuations of superparamagnetic particles thus intensifying an intermixing process, while constant magnets attract these particles to the walls of the chamber.

However, the above-described device has the following disadvantages;
A) It is highly energy consuming (due to high current consumption by alternating current coils);
B) It does not efficiently provide a continuous process of separation since it requires a periodic recurrence of the cycles of magnetic particles retaining with their subsequent releasing by means of removing constant magnets and washing out the particles from the walls of the chamber with a washing fluid;
C) It is not convenient for sterilization of the working chamber.

### Summary of Invention

It is a chief objective of the present invention to increase the reliability of simultaneous detection of a number of components in a mixture, to make the analysis cheaper and less time-consuming, to increase a range of indicators used and also to provide the possibility of visual registration of a number of components in a mixture.

Increasing the reliability of simultaneous detection of a number of components in a mixture, making the analysis cheaper and less time-consuming is also achieved due to providing a more efficient reacting of the mixture components with a minimum amount of microcarriers.

One more objective of the present invention is to develop an efficient and operation-saving separation of microcarriers in a convenient for sterilization separating chamber where the process of retaining the microcarriers on the walls of the separating chamber and removing them into the collector is actually continuous.

The objective set forth is achieved by a method for simultaneous detection of a number of components in a mixture consisting of reacting of the mixture components under analysis with the specific receptors immobilized on the surfaces of microcarriers of different forms. The formation of specific complexes causes the changes in the mobility of microcarriers under the influence of a physical field. These changes in the mobility of microcarriers under the influence of a physical field result in separation into a fraction of microcarriers which have not reacted and, hence, have not changed their mobility, and a fraction or fractions of microcarriers which have changed their mobility. Since each type of a specific receptor is bound to its marked microcarrier the analysis of just a marker characteristic of microcarriers with changed mobility under the influence of a physical field will reveal the availability of the component which has reacted with this microcarrier in the mixture under analysis. Under a physical field as in the case described a gravitation field, an electric field or a magnetic field as well as combinations of the above are understood.

The chief objective of the invention is achieved by a device for simultaneous detection of a number of components in a mixture, said device being comprised of a reacting chamber for reacting of the mixture components under analysis with the specific receptors immobilized on the surfaces of microcarriers, while each type of a specific receptor is bound to its marked microcarrier, and an analyzing apparatus is designed so that it additionally contains a separating chamber with the inlet of said separating chamber coupled to the outlet of a reacting chamber, said separating chamber being supplied with a means for generating a physical field for separation of microcarriers into those with non-reacted and those with reacted receptors respectively as judged by their mobility under the influence of a physical field, while the separating chamber is supplied with a means for delivery of separated microcarriers and/or information about the latter to the analyzing device.

A microcarrier can be produced from microscopic biological subjects made of synthetic, semi-synthetic or natural materials or mixtures of the above with microincapsulated markers coupled to a nucleus or a surface of microcarriers as well as from Protysta, bacteria, viruses, phages, spores of plants, bacteria and fungi, flower pollen of plants, erythrocytes, microscopic algae, magnetite, maghemite, iron, nickel, chrome, gold, silicagel, zeolite, carbon, polystyrene, divinyl benzene, polyacrylamide, polysulfone, polyamide, polyethylene glycol, glucans in α and β conformations, including aminoglucans, polyuronic acids, polyacrolein, polyglutaraldehide, polymethyl(hydroxymethyl)acrylate, polychloromethylsterene, polyvinyl alcohol, polyvinyl α-amino acids, polyvinyl isotronium, polyacrylic acid, polylactate, polyalkylcyarioacrylate or the mixtures and combinations of the above. Microcarriers used in one analysis have an identical shape with the dimensions differing by max. 20% and look like a sphere, a hemisphere, a cone, a polyhedron, a rod or a strand.

A microcarrier may be marked by a symbol or a relief on its surface which can be distinguished under a microscope as well as by a substance or a mixture of substances included into a microcarrier or affixed to its surface. Identifying features of a marker may be absorption spectra, emission spectra, reflection spectra, dispersion spectra, birefringence spectra, circular dichroism spectra or energy spectra of radioactive decay characteristic of the given substance or a mixture of substances as well as the intensity of said spectra. Organic complexes of cations of lanthanum group, natural and synthetic porphyrins, natural and synthetic quinones, natural and synthetic alkaloids, with paramagnetic markers and without, with radioactive markers and without may be used as markers as well as the mixtures of the above.

Characteristics of particles with varying mobility under the influence of a physical field are estimated either visually or by means of magnifying devices such as a microscope, a magnifying glass, a projector, a slide projector, a computer display or with the help of registering devices such as electromagnetic radiation spectrometers for microwave, infra-red, visible, ultra-violet and x-ray ranges respectively or for combinations of the above.

The second objective for a method for reacting of the components under analysis with the specific receptors immobilized on the surfaces of microcarriers, said method comprising a combination of microcarriers and a mixture in one chamber is achieved by providing an ordered motion of microcarriers one, two or more times through a medium containing a mixture under analysis. Besides it is possible to provide a counter motion of a medium containing a mixture under analysis.

Motion of microcarriers can be provided by a linear, circular or helical path.

Gravitation, electric or magnetic fields as well as combinations of the above can be used as a physical field. Motion of microcarriers in case of magnetic or electric fields can be provided by a mutual movement of a medium and a field.

The second objective in a reacting chamber for reacting of the components under analysis with the specific receptors immobilized on the surfaces of microcarriers made magnetic, said chamber comprising a mixture container and a means for generating a physical field in the form of at least one magnet, is achieved by making a mixture container as a channel, preferably a helical one, and by making a magnet with a possibility of its moving in relation to this channel. A helical channel may be formed by an elastic tube coiled up in the form of a helix, a baffle plate made on a cylindrical surface or on a disk plane in the form of a helix.

At least two magnets may be located on both sides of a helical channel with a shift in relation to one another.

A reacting chamber may additionally be supplied with a means for providing a counter motion of the mixture under analysis in relation to the motion of microcarriers and with additional fittings for inlet and outlet of microcarriers.

The third one of the objectives set forth for a separating chamber of microcarriers, said chamber comprising a means for generating a physical field in the form of at least one permanent magnet and a working chamber, inlet and outlet fittings for supplying a separated mixture and a washing fluid, is achieved due to a working chamber made of two removable cylindrical sterile cases (disposable ones) having a fixed connection to a permanent magnet.

Each case presents in itself a cylindrical cover with fittings having a groove into which a sealed disk is inserted with a possibility of rotating around its axis by means of a drive.

A drive is made as a frictional wheel which is contiguous to the surfaces of movable disks having the possibility of rotation in the counter directions with equal speeds.

A separating chamber is fixedly connected to a permanent magnet with the help of projections and grooves positioned on a magnet ring and on an internal surface of the cases.

A magnet is made in the form of a disk half with two built on parts having the form of the sectors truncated by 90° of half depth with a magnetic field orientation along the central axis of a disk.

Inlet and outlet fittings for a separated fluid are located at the level of the median of the case, while inlet fittings for a washing fluid are located at the top of the case and an outlet fitting for a washing fluid is located slightly above the level of the median of the case.

Each rotating disk in assembly with a cylindrical cover provides a self-contained volume separated by a baffle plate of a falculate shape formed on the interior part of a case cover and having a tight contact along its full length with a surface of a rotating disk.

An arch-shaped central part of the baffle plate forms a cavity, and its circumferential lengthwise part located radially at a small angle to the median forms an inclined channel along the bottom part of a rotating disk. In the bottom of the cavity thus formed there is installed a fitting for an outlet of a washing fluid with non-reacted microcarriers, and at the end of the inclined channel there is installed a fitting for an outlet of reacted microcarriers.

### Brief Description of Drawings

Fig. 1 shows a block diagram of a method for simultaneous detection of a number of components in a mixture as filed.
Fig. 2 shows a generalized block diagram of a device for simultaneous detection of a number of components in a mixture as filed.
Fig. 3 shows a principal diagram of a device for implementation of one of the versions of a method filed.
Fig. 4 shows absorption and emission spectra of dyes and markers of microcarriers according to the version of a method filed used in a device shown in Fig. 3.
Fig. 5 shows an example of implementation of a reacting chamber filed where the channel is made of an elastic tube coiled up in the form of a helix.
Fig. 6 shows a cross-sectional view by A-A of A device shown in Fig. 5.
Fig. 7 shows a version of a reacting chamber where the channel is formed by a baffle plate made on a cylindrical surface in the form of a helix.
Fig.8 shows a cross-sectional view by A-A of A device shown in Fig. 7.
Fig. 9 shows the constituent parts of a device shown in Fig.7.
Fig. 10 shows a version of a reacting chamber where the channel is formed by a baffle plate made on a disk plane in the form of a helix, and Fig. 11 shows a cross-sectional view by A-A of a device shown in Fig.10.
Fig. 12 shows implementation of a method filed for reacting in a reacting chamber shown in Fig.10 and Fig. 11. The items shown separately are as follows:
   a) A schematic picture of introduction of magnetic microcarriers into the chamber;
   B) A conditional path of ordered motion of magnetic microcarriers along a helical channel;
   c) A schematic picture of selecting the microcarriers from the chamber.

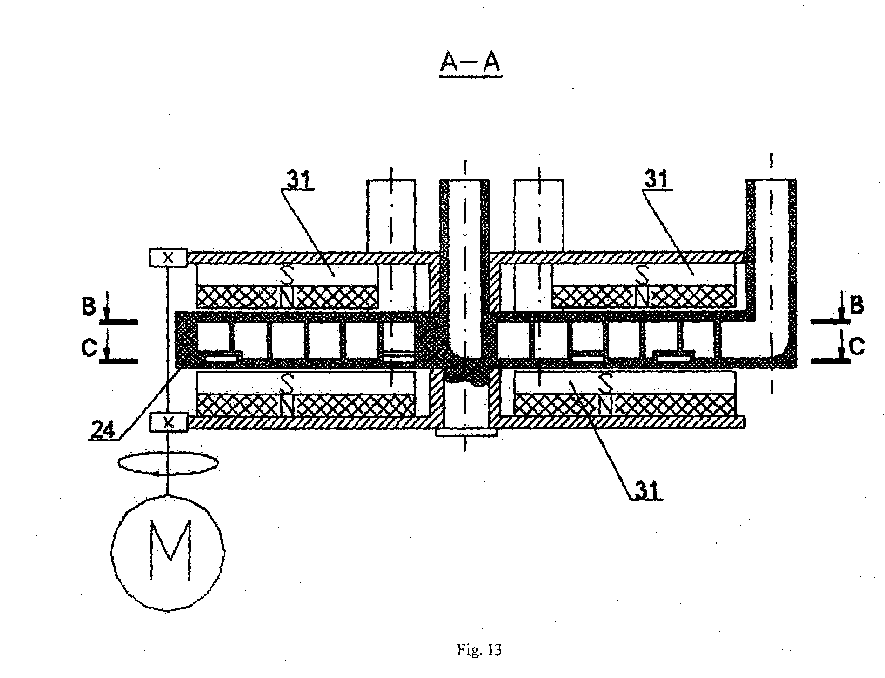

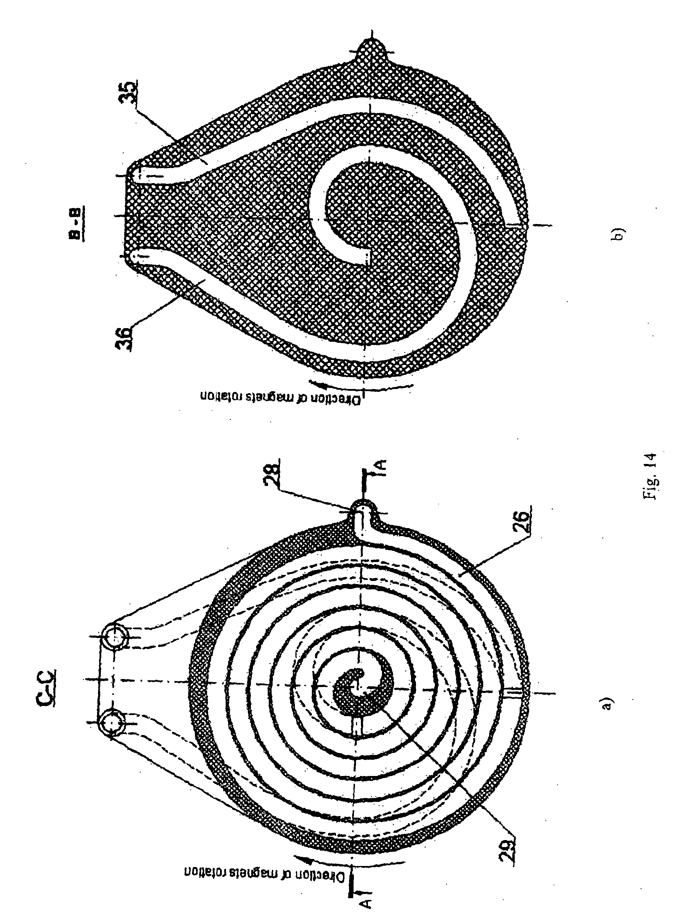

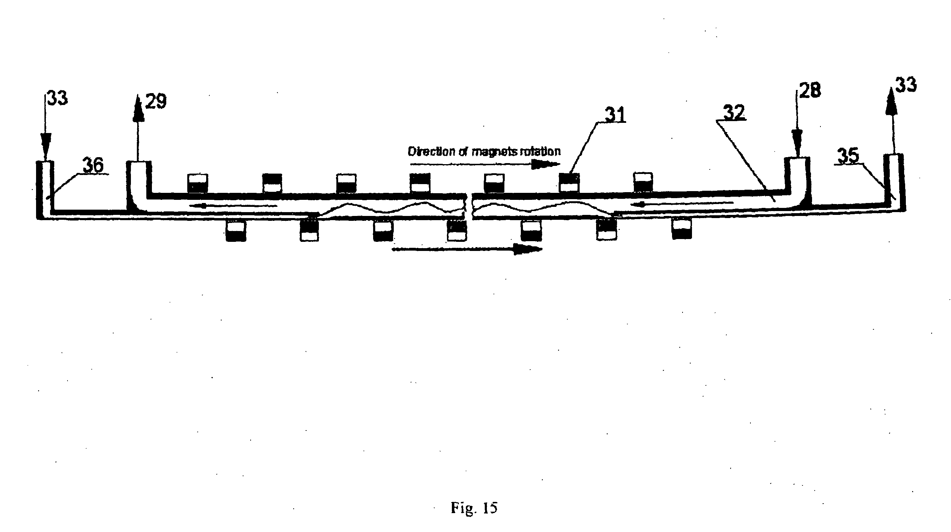

Fig.13 and Fig. 14 show a version of a reacting chamber where the channel is formed by a baffle plate made on a disk plane in the form of a helix, and a counter motion of a medium and a field is provided. Fig.13 shows a cross-sectional view by A-A, Fig.14a shows a cross-sectional view by C-C of the chamber described in a plane of a medium motion, and Fig.14b shows a cross-sectional view by B-B in a plane where the magnetic microcarriers are supplied and selected, and Fig. 15 shows an extended view of a helical channel with a conditional path of an ordered motion of magnetic microcarriers and a medium carrying a mixture under analysis in a version of a reacting chamber shown in Fig. 13 and Fig. 14.
Fig.16 shows a cross-sectional view of a separating chamber filed by A-A, Fig. 17 shows a cross-sectional view by D-D, Fig.18 shows a cross-sectional view by C-C, Fig.19 shows a cross-sectional view by B-B.

### Detailed Description of Invention and Preferable Examples

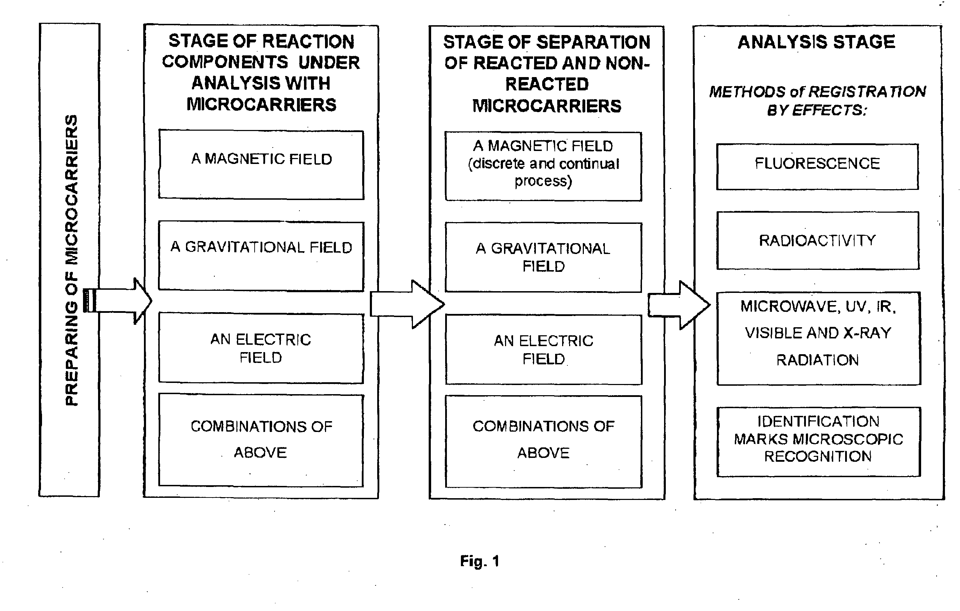

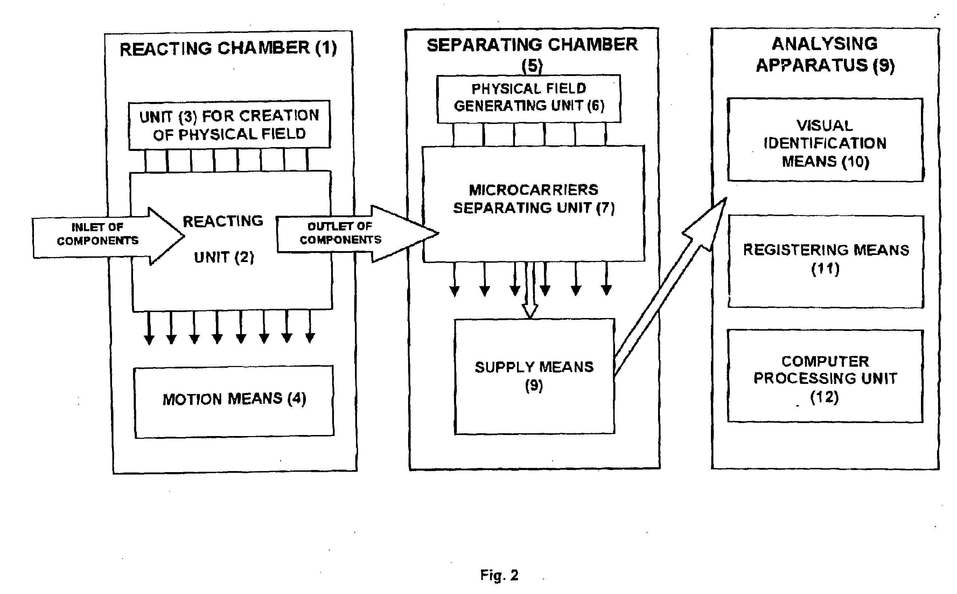

A step-by step description of a method filed is given with reference to Fig. 1 and Fig. 2.

Firstly the microcarriers are prepared.

At this stage pre-produced microcarriers of one type (for example, positively or negatively charged, magnetically controlled or not) with a deviation of parameters by max. 20 % are divided into portions, with the number of said portions being equal to a proposed number of components to be detected in a mixture. On the surfaces of microcarriers of each of said portions there is applied a pre-defined marker and a specific receptor is immobilized thereon.

Then the aliquots comprising from 1 to 1000 microcarriers, preferably 3-5 microcarriers, are selected from each of said portions.

Further the aliquots of microcarriers of each type are mixed up.

Then the reacting of the mixture components under analysis with prepared microcarriers is carried out. Reacting is provided in a reacting chamber 1 (see. Fig. 2).

In the least sophisticated case the reacting is provided by combination in one chamber (in reacting unit 2) of the medium comprising a mixture under analysis and prepared microcarriers.

In more preferable cases a physical field is additionally applied by means of unit 3 for generating a physical field to optimize a reacting process. It is possible to provide an additional motion of the mixture under analysis with the help of respective means 4.

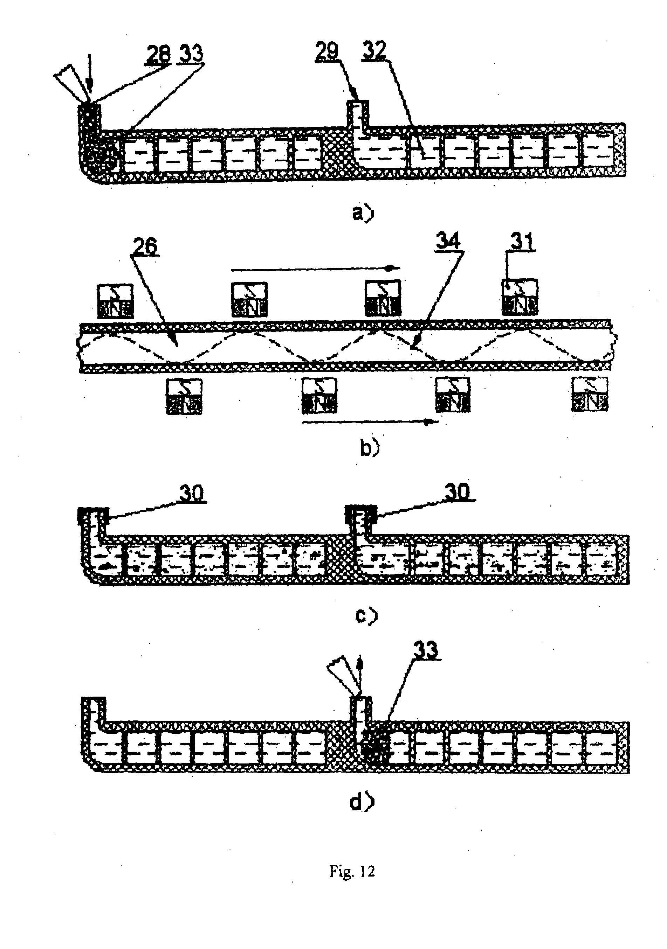

In its best way the reacting can be provided by means of a method filed which is shown in a diagrammatic form in Fig. 12. According to this method the microcarriers thus prepared are influenced by a physical field in such a way that their ordered motion through the channel filled by said medium is provided. For elongation of a microcarrier path the channel may be made linear, helix or circular , while the motion of microcarriers through the channel can be provided for a number of times.

Moreover the counter motion of said medium can also be provided.

A more detailed disclosure of a method of reacting filed will be given during description of respective devices.

During reacting the mixture components are bound to specific receptors immobilized on a surface of respectively marked microcarriers.

The following stage is the separation of microcarriers into those with reacted and those with non-reacted receptors. This stage is carried out in separating chamber 5 (Fig. 2).

For this purpose all mass of microcarriers is started to motion under the influence of a physical field from unit 6 which may be a magnetic field, an electric field, a gravitation field or combinations of the above.

The components which have bound during reacting cause the changes in the dimensions and/or in a charge, and/or in a magnetic susceptibility of microcarriers, this in its turn resulting in the changes of their mobility under the influence of a physical field.

During the motion under the influence of a physical field in separating unit 7 the microcarriers are separated in two groups: those which have reacted and those which have not reacted. Depending on the type of a field the microcarriers which have reacted will either advance or lag behind as compared to those which have not reacted.

After selecting the desired group of microcarriers the latter and/or information about them are supplied to analyzing means 9 with the help of supply means 8.

The markers available in a selected group of microcarriers are identified. The identification is carried out depending on the marker type either visually (means 10) or with the help of the instruments (means 11).

According to the markers which have been identified a conclusion is made about the components which are available in the mixture (should the microcarriers which have reacted be analyzed) or about the components which are not available in the mixture (should the microcarriers which have not reacted be analyzed). Processing and storage of the data thus received is provided in processing unit 12 which may be a computerized one.

The implementation of a method filed can be illustrated by the following non-limiting examples.

### Example 1.

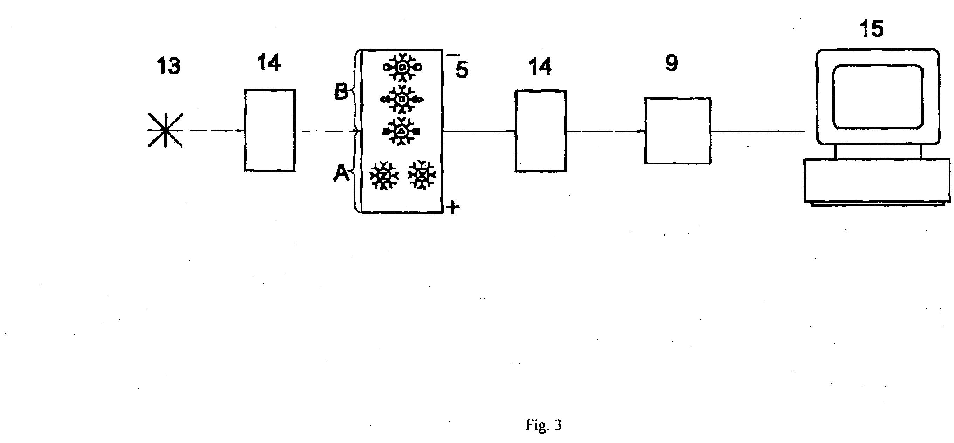

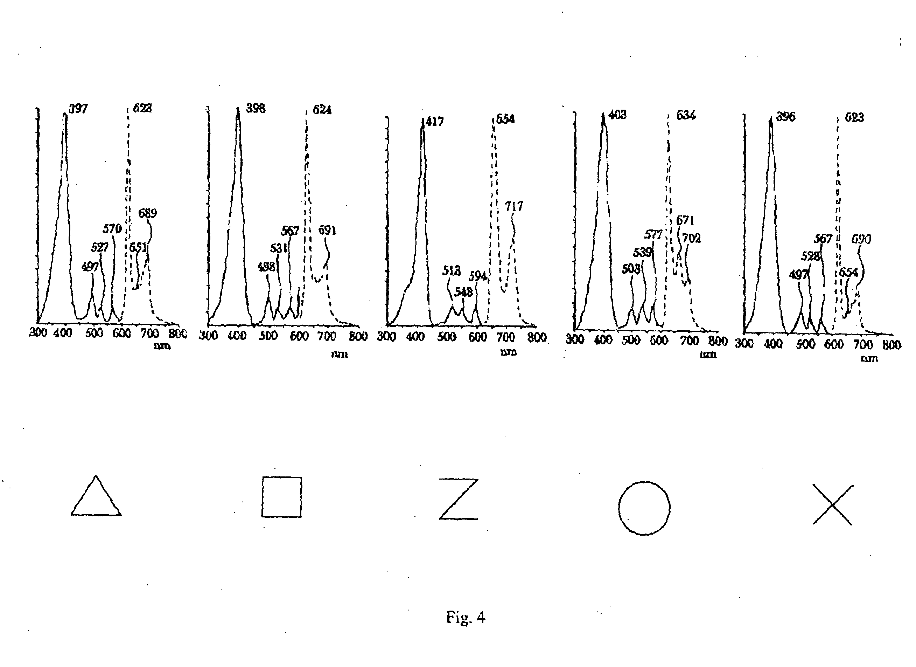

Fig.3 shows a principal diagram of a device filed for implementation of one of the versions of a method filed. An ultraviolet radiation source 13, optical filters 14 and analyzing means 9 which is a fluorescence detector are installed on an optical axis together with separating chamber 5 in the form of a capillary electrophoresis apparatus (not shown at the drawings). Active fluorescent dyes N 33258, N 33276, N 33285, N 33514 and N 33258 with absorption and emission spectra shown in Fig.4 were used for encapsulation of microcarriers with the spheres made of chitin having the dimensions of 2 Mkm. Each type of marked microcarriers thus obtained was combined with monoclonal antibodies for the following human's blood serum proteins (albumin, transferrin, fibrinogen, cerulloplasmin and α2-microglobulin) by means of reaction with carbodiimide. The complexes thus obtained were fractionated in an electric field in a capillary electrophoresis apparatus with an electric field intensity of 30 V/cm in 0,1 M Tris-glycine buffer pH 8,3. Fractions with identical mobility were combined in a test system such that to provide equal proportions of each microcarrier in a mixture. Tests systems aliquots of 5 Mkg of spheres as estimated were added to the samples of blood serum in volume 0,1 ml. After reacting during incubation in a thermostat at the temperature of 30°C and stirring in a separate test tube (not shown) during 30 minutes the samples were fractionated in an electric field in separating chamber 5 in a capillary electrophoresis apparatus with an electric field intensity of 30 V/cm in 0,1 M Tris-glycine buffer pH 8,3. During this step a separation of microcarriers into two clearly discernible fractions was observed: one with unchanged mobility (designated as A zone in Fig. 3) and "tail" B of microcarriers with decreased mobility. These microcarriers were lighted by UV radiation from source 13 through optical filters 14. Analyzing means 9 (a fluorescence detector) was used to register absorption and emission spectra of microcarriers' markers with changed mobility. Computerized system 15 was used to identify the availability of albuminum, transferrin, fibrinogenum, cerulloplasmin and α2- microglobulin in accordance with indicated spectra in the samples analyzed.

### Example 2

Microcarriers in the shape of needle crystals having the dimensions of 3x0,2 mkm made of synthetic zeolites of H-6, K-4, M-8 and KO-4 brand were covered by a thin layer of dibutyrilchitin with the fragments of pollen of the following cereals added to it, i.e. rye, wheat, oats and fowl distinguished by their characteristic shape and visible light polarization. Each type of marked zeolite was pre-fractionated in an electric field of a capillary electrophoresis apparatus with an electric field intensity of 30 V/cm in 0,1 M Tris-glycine buffer pH 8,3. Fractions with identical mobility were combined in a test system to provide equal proportions of each zeolite in a mixture.

The mixture under analysis of K, Na, Cu and Co cations in the form of 0,1 mkM solutions in the amount of 0,2 ml was added to the test system, and re-separation was carried out in a capillary electrophoresis device with an electric field intensity of 30 V/cm in 0,1 M Tris-glycine buffer pH 8,3. Fractions of microcarriers with decreased mobility under the influence of an electric field were viewed in a polarized light under a microscope. On the basis of visual evaluation of a microscopic image a conclusion was made about the availability of individual cations in the mixture under analysis.

### Example 3.

The suspension of bacteria *Magnetospirilla gryphiswaldense* with the dimensions of 42x5 mkM was mixed with a 6% water solution of polyvinyl alcohol, and a 1% water solution of glutaraldehyde was added at intensive stirring. After washing out of non-reacted components and water-soluble reaction products the particles thus received were chemically bound with active fluorescent dyes N 33258, N 33276, N 33285, N 33514 and N 33258 with absorption and emission spectra of said dyes shown in Fig.4. Each type of marked magnetically responsive microcarriers was bound with avidin molecules by means of tosylation. Biotinilated monoclonal antibodies were bound to the following human blood serum proteins such as albumin, transferrin, fibrinogen, cerulloplasmin and α-2- microglobulin by means of incubation of each type of antibodies with microcarriers marked with a definite dye. The complexes thus obtained were pre-fractionated in an CTV (cell tracking velocimetry) apparatus for detection of magnetophoretic mobility of cells or particles wherein the use was made of a strictly controlled magnetic field gradient, said apparatus comprising a microscopic imaging device and a computer program for detection of magnetophoretic mobility of an appreciable amount of particles (>200) and a means for their separation into individual fractions. Fractions with identical mobility were combined in a test system to provide equal proportions of each type of microcarriers in a mixture. Test system aliquots of 5 mkg of spheres as estimated were added to the samples of blood serum in volume of 0,1 ml, and after stirring and incubation during 30 minutes the samples were re-separated in a CTV apparatus. The availability of albumin, transferrin, fibrinogen, ceruloplasmin and α-2-microglobulin in the samples under analysis was identified on the basis of absorption and emission spectra of microcarriers with changed mobility.

### Example 4.

Spheres of polystyrene of 5±1 mkm were coloured by incubation of samples of 10 Mg in 0,1% benzene solutions of the following dyes: active bright-red 5CX, active bright-yellow 53, active orange 2G, active bright-blue K. Addition of antibodies to groupspecific antigens and a Rh factor of human blood was carried out by a nonspecific sorption in sodium - phosphate buffered solution (SPB) pH 7,4. Spheres of each group after bonding of antibodies were pre-fractionated by decantation during 30 minutes. A test system was formed out of fractions with identical rate of sedimentation in a gravitation field. Erythrocytes ghosts from the blood samples under analysis in volume of 0,2 ml were received by dilution of samples with distilled water in a proportion of 1:9 and centrifugation at 3000g during 15 minutes. Test system aliquots of 5 mkg of spheres were added to precipitate of the erythrocytes ghosts, stirred and applied on a surface of SPB layer of 5 cm in height in glass test tubes. Separating of spheres into two fractions was observed in 15 minutes. The fraction precipitating with high speed contained the spheres which have reacted with erythrocytes, and judging by a visually determined colour of this fraction a conclusion was made about erythrocytes being attributed to a definite blood group and a Rh factor.

### Example 5

Experimental conditions were as in Example 4 except that for reacting of the erythrocytes ghosts with microcarriers the stained polystyrene spheres of 5 mkg were used, and reacting was provided by layering the mixture of spheres in a fluid layer containing a suspension of the erythrocytes ghosts with the height of 10 cm in a glass tube having the diameter of 0,2 mm and by double passage of the spheres through the whole layer under the influence of a gravitation field. Microcarriers collected after reacting were separated by precipitation in a gravitation field in a layer of a physiological solution with the height of 5 cm and diameter of 0,2 mm. A distinct separation of reacted and non-reacted microcarriers was observed, thus providing the basis for the conclusion about the attribution of erythrocytes to a certain blood group and a Rh factor.

### Example 6

Spores of a fungus *Asperigillus niger* were covered with magnetite in a polystyrene layer by means of emulsion polymerization. For this purpose 2 g of dry spores were added with 4 ml of a mixture of styrene, 40% of a magnetite suspension in styrene, a methacrylic acid and divinyl benzene in the ratio of 3:2:0,3, 1 ml and carefully stirred. A mixture thus obtained was added to 50 ml of 0,1% aqueous solution of sodium dodecylsulphate and further ultrasonically treated with the intensity of 50% during 5 minutes. The emulsion thus obtained was added with 250 Mg of sodium persulphate and incubated during 3 hours at the temperature of 60°C at constant stirring. Then a suspension was filtrated through a dense cotton fabric and washed from the oddments of non-reacted monomers by means of a membranous filtration on hollow polysulphone fibers with the porosity of 500 kD. Magnetically responsive microcarriers thus obtained in aliquots of 0,1 mg were added separatly in concentration of 2mkg/ml with fluorescent dyes such as Nº33258, Nº33276, Nº33285 Nº33514 and Nº33258 with absorption and emission spectra of said dyes shown in Fig.4. After incubation with alcohol solutions of fluorescent dyes the microcarriers were water washed to remove a dye excess with the help of magnetic retaining. Marked microcarriers were further used for detection of serum proteins in a human's blood: albumin, transferrin, fibrinogen, cerulloplasmin and α-2-microglubulin as it is described in Example 3.

### Example 7

Experimental conditions were the same as in Example 6 except that instead of spore *Asperigillus niger* a culture *of Streptococcus acidophylus* was used.

Implementation of a method and a reacting device filed is described in the examples that follow.

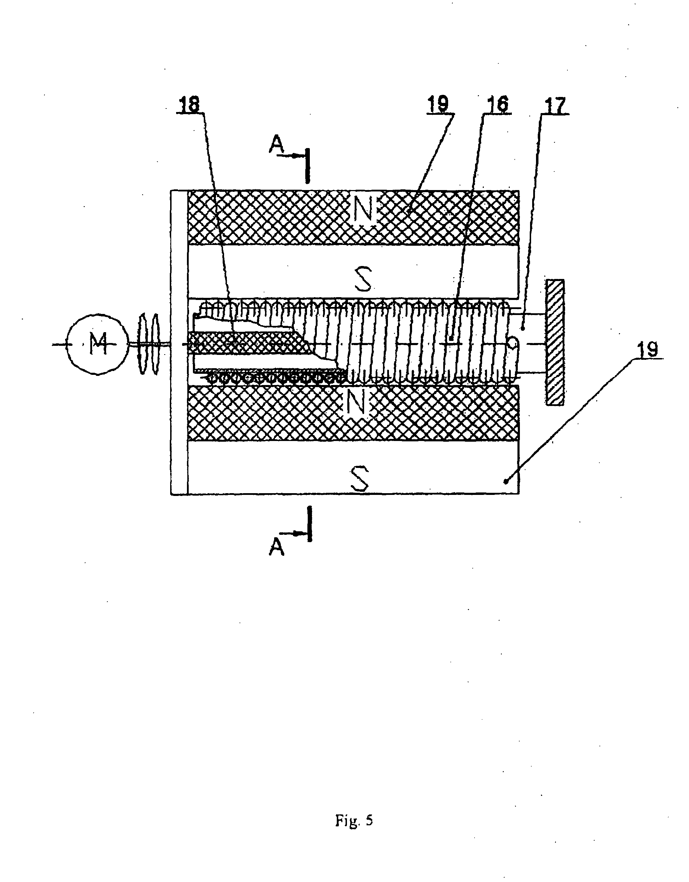

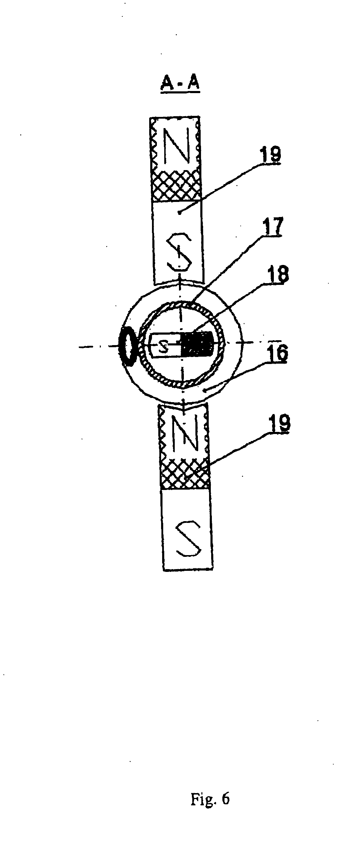

Reacting chamber 1 as filed in its least sophisticated version shown in Fig. 5 and Fig. 6 is comprised of elastic tube 16 coiled up in the form of a helix around hollow cylinder 17. Permanent magnet 18 of 1 plate-like structure is installed inside cylinder 17, and outside cylinder 17 there are installed two permanent magnets 19 closely adjoining the turns of a helix with an orthogonal orientation of their magnetic field gradient in relation to a field gradient of internal magnet 18.

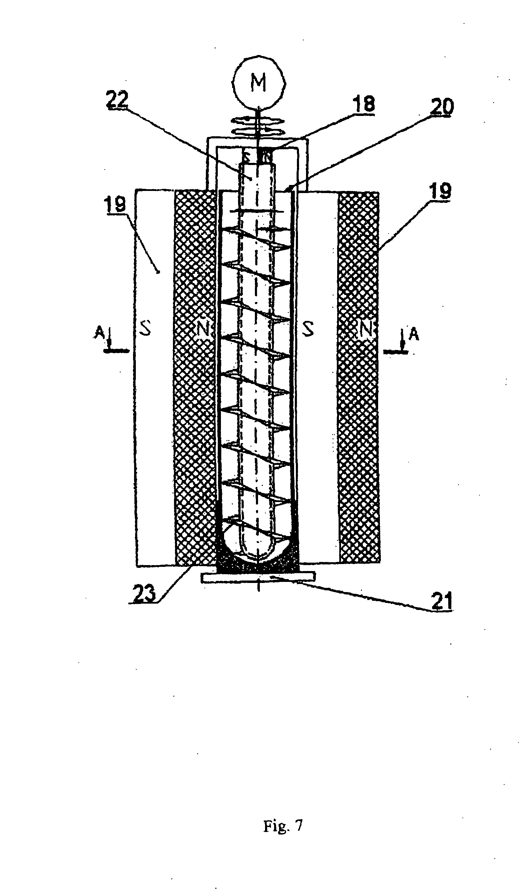

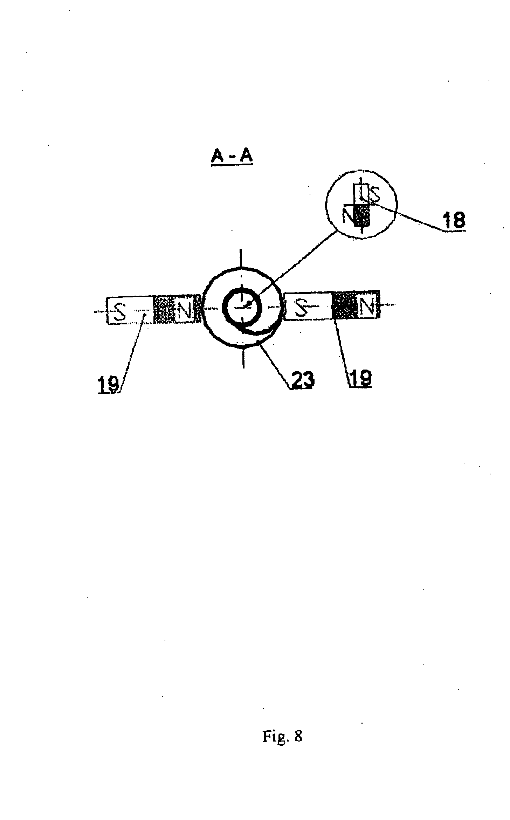

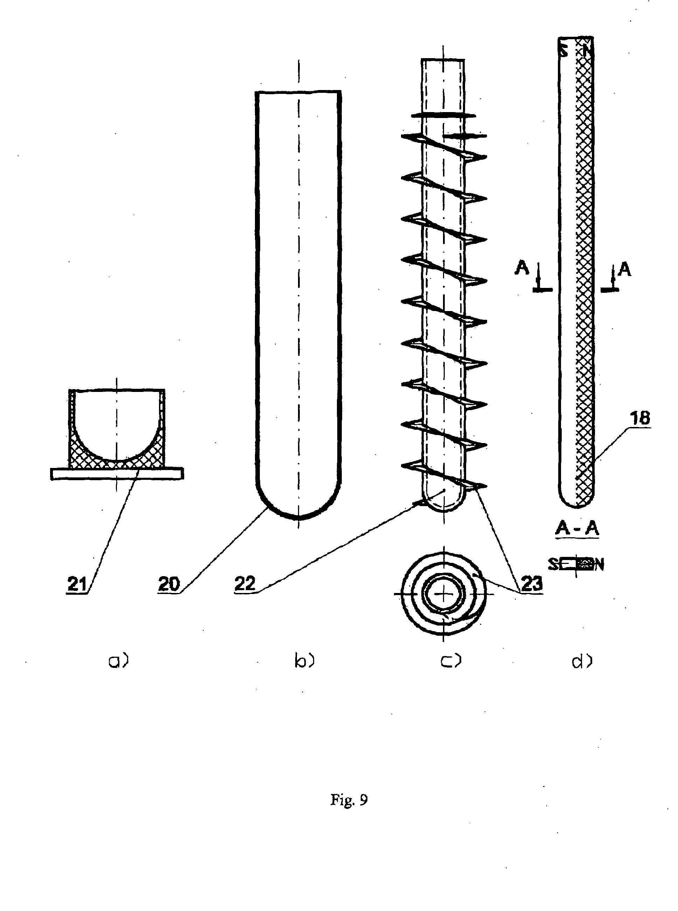

A reacting chamber according to the version shown in Fig. 7, Fig. 8 and Fig. 9 is comprised of test tube 20, test tube holder 21, insert 22 with helix baffle plate 23 and a system of internal magnet 18 and external magnets 19 with an orthogonal orientation of magnetic field gradient of the latter in relation to a field orientation of internal magnet 18. Fig. 9 shows the above mentioned component parts of the described version of a reacting chamber as filed.

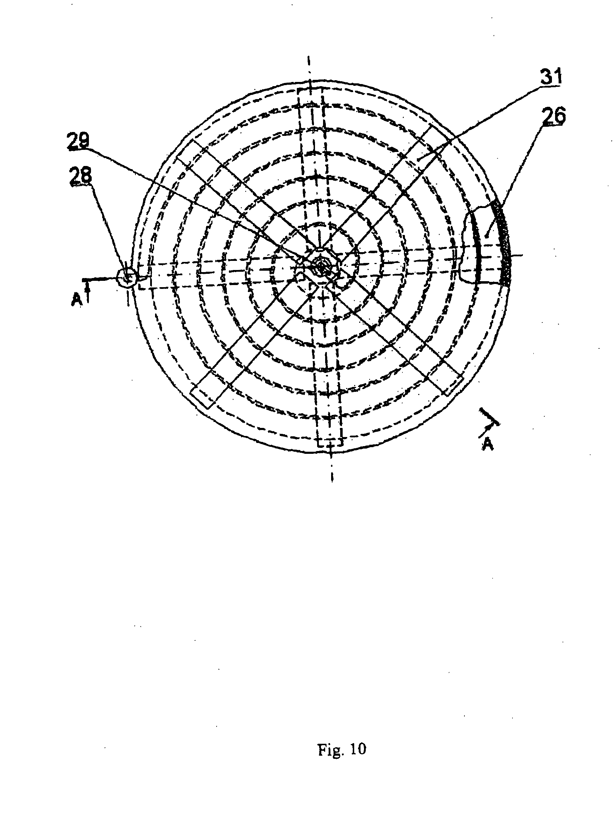

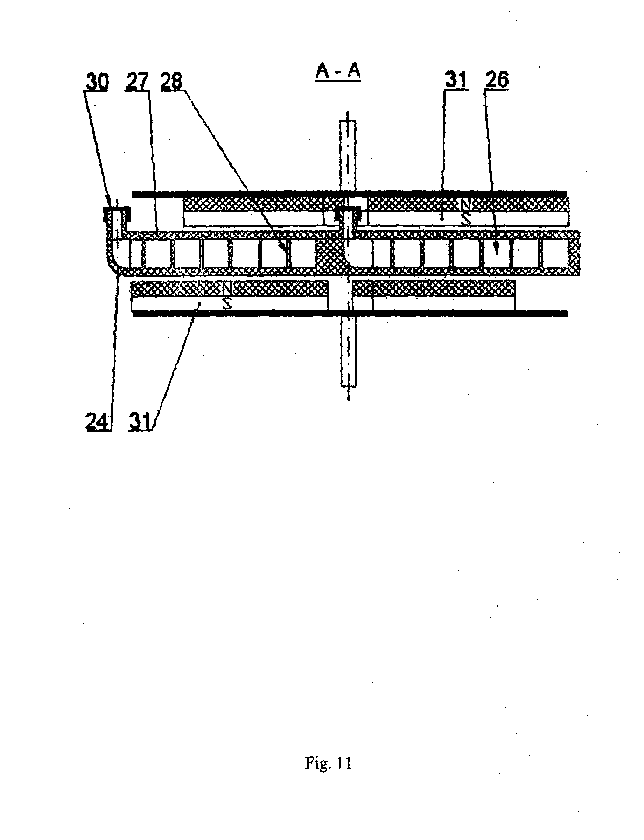

A device according" to the version shown in Fig 10 and Fig. 11 is comprised of case 24 with helix baffle plate 25 forming channel 26 with cover 27, said cover provided with inlet 28 and outlet 29 openings having plugs 30, and a magnetic system. The magnetic system in its turn is comprised of radial positioned permanent magnets 31 installed on both sides of case 24 with a shift in relation to one another.

The version of implementation of a method in a device according to Fig. 10 and Fig. 11 is shown in Fig. 12. After filling channel 6 with medium 32 containing the mixture under analysis plugs 30 of the openings are removed. Magnetic microcarriers 33 are applied in the form of a concentrated narrow zone in the area of inlet opening 28 with openings 28 and 29 closed by plugs 30 afterwards. During rotation of magnets 31 around the axis with a definite speed the microcarriers 33 drawn by magnets 31 undergo the resistance to their motion due to viscosity of medium 32, this resulting in their periodic tearing off and passing under the influence of shifted magnet 31 on the opposite side, thus providing a sine wave path 34 of their motion through said medium. This makes it possible to achieve a maximum efficient bonding of microcarriers with the components under analysis in a medium volume on the whole. Magnets 31 are positioned on both sides of helical channel 26 with a shift in relation to one another.

Reacting chamber 1 with a mixture under analysis constantly flowing through it is shown in Fig.13 and 14. The chamber is comprised of case 24 having inside channel 26 for medium 32 containing the mixture under analysis, said channel made in the form of a flat helix and supplied with inlet 28 and outlet 29 openings, said inlet 28 being located on a circumference of the reacting chamber and coupled with a means for providing motion of medium 32 e.g. a pump (not shown) for supplying medium 32 to channel 26 and outlet opening 29 located in the center and coupled with a tank for medium collection. Initial part of channel 26 at some distance from inlet opening 28 is adjoined by exhaust channel 35 of smaller cross section for exhausting the suspension containing paramagnetic microcarriers 33. An ultimate part of channel 26 at some distance from outlet opening 29 is adjoined by supply channel 36 of the same cross section for supplying the suspension containing both reacted and non-reacted paramagnetic microcarriers.

Outlet channel 35 and inlet channel 36 are located under channel 26 in a plane parallel to it and have a specific orientation of the helix fragments, thus providing a required motion direction of a suspension containing paramagnetic microcarriers under rotation of magnets 31.

Paramagnetic microcarriers 33 are constantly fed to supply channel 36 by means of a pump (not shown) and further under the influence of a magnetic field gradient of rotating magnets 31 are introduced into channel 26 and counter moved in relation to a flow of the mixture under analysis. After passing through channel 26 in full microcarriers 33 are removed from suspension by means of exhaust channel 35 and also under the influence of a magnetic field gradient of rotating magnets 31. Effective operating zone of lower magnets is spread closer to the center of helix channel, thus providing a preferential motion of paramagnetic microcarriers on the last turns of a helix along the lower wall of channel 26 which is directly adjoined by inlet of suspension exhaust 35.

The efficiency of a reacting method filed (see Fig. 12a-c) in one of the implementations of a device filed is illustrated by the following non-limiting examples.

### Example 8

Helical channel 26 of a device shown in Fig. 10-11 was filled with 10 ml of standard water solution of HgCl₂ in concentration of 10⁻⁶ g/l. Then 10 mkl of 1 % suspension of microcarriers 33 of magnetite (Fig. 12a) stabilized by chitosan was introduced through an inlet opening. Inlet 28 and outlet 29 openings were plugged in with plugs 30 and a system of magnets 31 was started to rotate with the speed of 2 min⁻¹. The rotation was stopped in 10 minutes after microcarriers 33 of magnetite had gathered at outlet opening 29. After removing plugs 30 and magnets 31 the microcarriers were carefully collected by a pipette in the volume of 50 mkl (Fig. 12c). After ashing of the sample a quantification analysis of mercury content was carried out by a method of atom adsorption spectrometry in Varian AAS equipment. Mercury in the amount of 8,92·10⁻⁹g was defined in the sample.

Simultaneously a sample of standard water solution of HgCl₂ in concentration of 10⁻⁶ g/l in volume of 10 ml was evaporated during 1 hour in a dryer at 120°C, and the analysis was carried out in Varian AAS equipment. Mercury in the amount of 8,66·10⁻⁹g was defined in the sample.

### Example 9

Experiment conditions were the same as in the previous example except for the concentration of

HgCl₂ solution which was 10⁻⁷ g/l. Mercury in the amount of 0,91•10⁻⁹g was defined in the sample as a result of reacting with mercury ions by a method filed, while mercury in the amount of 0,96·10⁻⁹g was defined in the control sample obtained by evaporation.

As it can be seen from the examples the reacting by a method filed provided in one of implementations of a device filed makes it possible to extract the components desired from a mixture practically completely.

A stage of separation of reacted and non-reacted microcarriers may be implemented in a separating chamber filed.

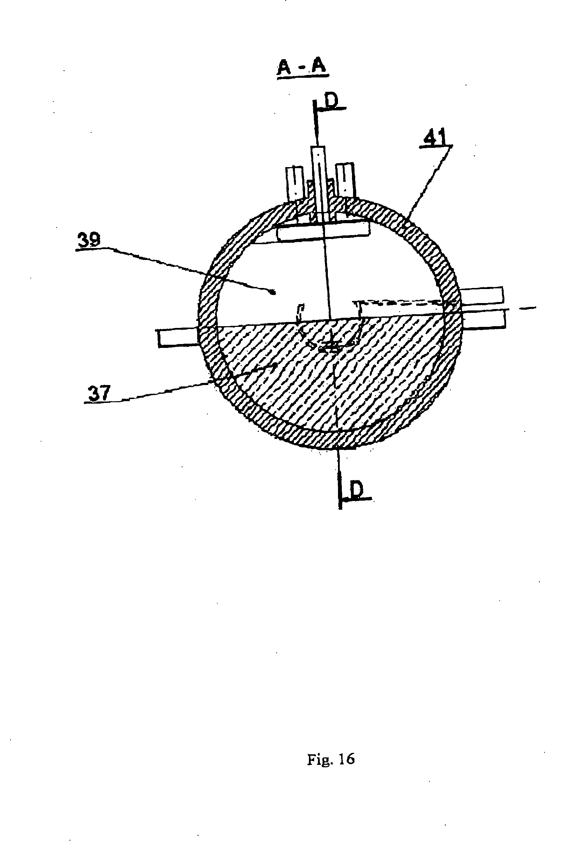

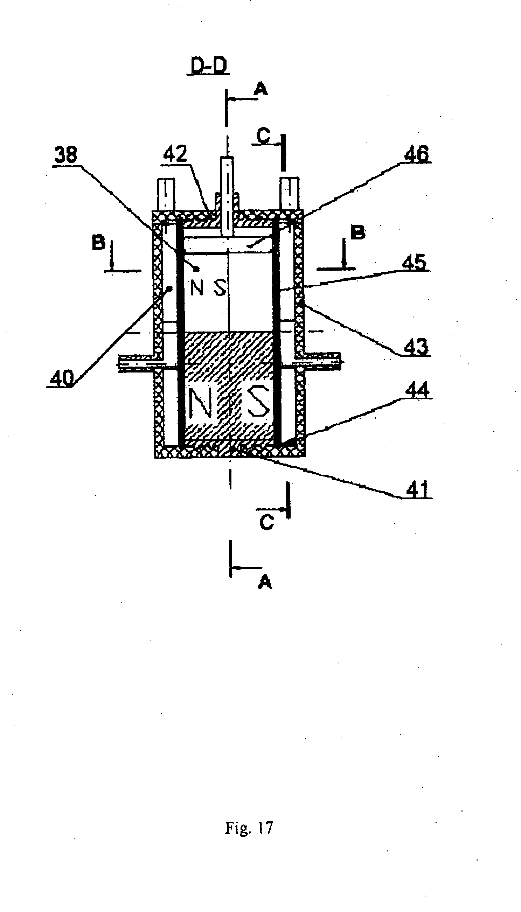

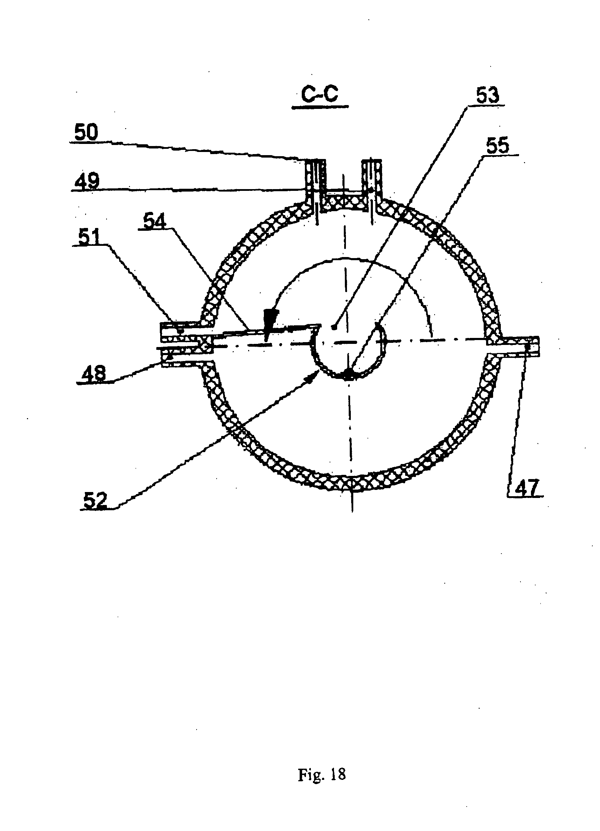

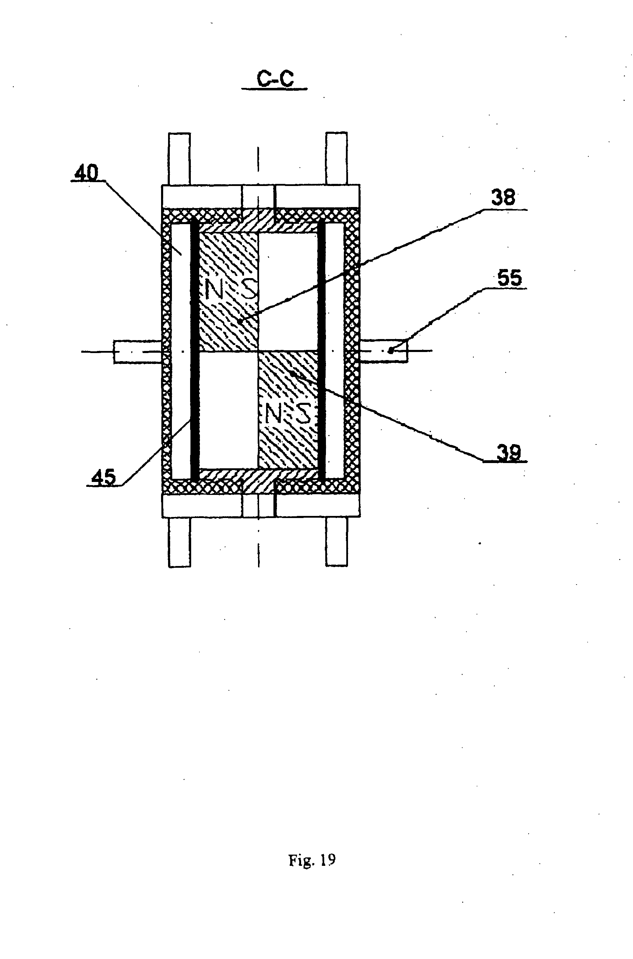

Separating chamber 5 as filed which is shown in Fig. 16,17,18,19 is comprised of a permanent magnet made in the form of a disk 37 half with two built on parts made in the form of sectors 38 and 39 truncated by 90° of half depth with a magnetic field orientation along the central axis of disk 37 and two identical cases 40 joined with magnet ring 41 by means of projections and grooves 45 located on the ring and on the internal surface of cases 40. Each case presents in itself cylindrical cover 43 having a groove 44 into which sealed disk 45 is inserted with the possibility of rotation around its axis.

A drive for rotation of disks 45 is made in the form of frictional wheel 46 contiguous with the surfaces of movable disks 45. Wheel 46 is coupled to an electric motor (not shown in the drawings). Inlet 47 and outlet 48 fittings for separated mixture are located at a median level of the case, and fittings 49 and 50 for washing fluid supply are located at the top of case 40. Fitting 51 for washing fluid exhaust is located slightly above a median level of the case. The rotation of wheel 46 causes disks 48 to rotate in counter directions with identical speeds. Rotating disk 45 in assembly with cylindrical cover 43 forms a self-contained volume separated by baffle plate 52 of falculate form executed on the internal part of cover 43 of case 40. Baffle plate 52 has a tight contact along its full length with a surface of rotating disk 45. An arch-shaped central part of baffle plate 52 forms cavity 53, and a circumferential direct part located radially under a small angle to the median forms inclined channel 54. In the bottom part of cavity 53 thus formed there is installed fitting 55 for exhaust of washing fluid with the oddments of non-reacted components of separated fluid, and at the end of an inclined channel there is installed fitting 14 for removal of magnetically responsive particles with reacted components thereon. Separated fluid flows along the interior half of rotating disk 45, and washing fluid is supplied in two flows through fittings 49 and 50 to the top half of rotating disk 45. A fluid flow through fitting 49 removes the oddments of non-reacted components of separated fluid to a cavity 53 and fitting 55. During passing of separated fluid through working chambers magnetically responsive microcarriers are attracted under the influence of a constant magnetic field gradient to an internal surface of rotating disks 45 and are removed from a flow of separated fluid. The fluid purified from microcarriers is supplied to the receiving tank (not shown in the drawings) through outlet fitting 48. Owing to rotation of disk 45 the microcarriers reach a position out of the influence of a constant magnetic field gradient and are retained on a surface of disk 45 out of the flow of separated fluid only due to adhesion forces. Under the influence of the flow of washing fluid coming through fitting 50 weakly attached particles are washed out to an inclined part of falculate baffle plate 52 and are exhausted from the separating chamber.

The scope of the invention filed is not limited by the examples described.

## Claims

1. A method for simultaneous detection of multiple components in a mixture, said method comprises of reacting of the mixture components under analysis with the specific receptors immobilized on the surfaces of microcarriers with each type of specific receptor bound to its marked microcarrier, wherein on completion of reacting the separation of microcarriers into those with reacted and those with non-reacted receptors as judged by their mobility under the influence of a physical field is provided, and availability or non-availability of the components under analysis in a mixture is detected by identification of the markers of separated microcarriers.

2. A method of claim 1, wherein a physical field is a gravitation field, an electric field, a magnetic field or combinations of the above.

3. A method of claim 1, wherein a microcarriers are produced from microscopic biological subjects, synthetic, semi-synthetic or natural substances or mixtures of the above with microincapsulated markers bound with a nucleus or a surface of said microcarriers.

4. A method of claim 3, wherein a microcarriers are produced from Protysta, bacteria, viruses, phages, spores of plants, bacteria and fungi, flower pollen of plants, erythrocytes, microscopic algae, magnetite, maghemite, iron, nickel, chrome, gold, silicagel, zeolite, carbon, polystyrene, divinyl benzene, polyacrylamide, polysulfone, polyamide, polyethylene glycol, glucans in α and β conformations, including aminoglucans, polyuronic acids, polyacrolein, polyglutaraldehide, polymethyl(hydroxymethyl)acrylate, polychloromethylsterene, polyvinyl alcohol, polyvinyl α-amino acids, polyvinyl isotronium, polyacrylic acid, polylactate, polyalkylcyanoacrylate or mixtures and combinations of the above.

5. A method of claim 3, wherein the microcarriers used in one analysis have identical shape, and their dimensions differ by max. 20%.

6. A method of claim 3, wherein a shape of microcarriers looks like a hemisphere, a cone, a polyhedron, a rod or a strand.

7. A method of claim 3, wherein a microcarriers are marked by a symbol or a relief on their surface viewed under a microscope.

8. A method of claim 3, wherein a microcarriers are marked by a substance or a mixture of substances included in said microcarriers or affixed to their surface.

9. A method of claim 8, wherein the identifying features of a marker are an absorption spectra, an emission spectra, a reflection spectra, a dispersion spectra, a birefringence spectra, a circular dichroism spectra or an energy spectra of radioactive decay characteristics of a given substance or a mixture of substances as well as the intensity of said spectra.

10. A method of claim 8, wherein the markers are organic complexes of cations of lantanum group, natural and synthetic porphyrins, natural and synthetic quinones, natural and synthetic alkaloids with/or without paramagnetic markers, with/or without radioactivity as well as mixtures of the above.

11. A method of claim 1, wherein the characteristics of particles with changed mobility under the influence of a physical field are evaluated either visually or by means of registering means.

12. A method of claim 11, wherein the registering means are the spectrometers of electromagnetic radiation in radio, infra-red, visible and X-ray ranges taken as individually so in combinations of the above.

13. A method of claim 11, wherein the evaluation is provided either visually or with the help of magnifying devices.

14. A method of claim 13, wherein a microscope, a magnifying glass, a projector, a slide projector or a computer display are used as magnifying devices.

15. A method for reacting of the components under analysis with the specific receptors immobilized on the surfaces of microcarriers, said method comprising a combination of microcarriers and a mixture in one chamber, wherein an ordered motion of microcarriers through a medium containing the components under analysis is provided.

16. A method of claim 15, wherein a counter motion of a medium containing the components under analysis is provided.

17. A method of claims 15 or 16, wherein a motion of microcarriers is provided by a linear path, a circular path or a helix path.

18. A method of any of claims 15 - 17, wherein a motion of microcarriers is provided under the influence of a physical field.

19. A method of claim 18, wherein a physical field is a gravitation field, an electric field, a magnetic field or combinations of the above.

20. A method of claim 19, wherein a physical field is a magnetic field or an electric field, and a motion of microcarriers is provided under conter movement of a medium and a field.

21. A method of claim 20, wherein the microcarriers are passed two and more times through the whole volume of a medium under analysis.

22. A device for simultaneous detection of multiple components in a mixture, said device comprised of a reacting chamber for reacting of the mixture components under analysis with the specific receptors immobilized on the surfaces of microcarriers, while each type of a specific receptor is bound to its marked microcarrier, and of an analyzing means, wherein said device is additionally supplied with a separating chamber with the inlet of said chamber coupled to the outlet of a reacting chamber, said separating chamber supplied with a unit for generating a physical field for separation of microcarriers into those with non-reacted and those with reacted receptors as judged by their mobility under the influence of a physical field, while a separating chamber is equipped with a means for supplying the separated microcarriers and/or the information about the latter to an analyzing means.

23. A reacting chamber for the mixture components under analysis with the specific receptors immobilized on the surfaces of microcarriers, with the latter made paramagnetic, said reacting chamber comprising a mixture container and a unit in the form of at least one magnet for generating a physical field, wherein a mixture container is made in the form of a channel, and a magnet is made with the possibility of movement in relation to said channel.

24. A reacting chamber of claim 23, wherein a channel is made in the form of a helix.

25. A reacting chamber of claim 23, where a channel is made of an elastic tube coiled up in the form of a helix.

26. A reacting chamber of claim 23, wherein a channel is formed by a baffle plate made in the form of a helix on a cylindrical surface.

27. A reacting chamber of claim 23, wherein a channel is formed by a baffle plate made in the form of a helix on a disk plate.

28. A reacting chamber of claim 23, wherein at least two magnets are positioned on both sides of a helix channel with a shift in relation to one another.

29. A reacting chamber of any of claims 23-28, wherein said chamber is additionally supplied by a means for supplying a motion to a mixture under analysis in a counter direction in relation to a motion of microcarriers, and it is supplied with fittings for supply and collection of microcarriers.

30. A separating chamber comprising a unit for generating a physical field in the form of a permanent magnet and a working chamber, inlet and outlet fittings for a mixture under analysis and a washing fluid, wherein said working chamber is made of two removable sterile cylindrical cases which are disposable ones, said cases fixedly secured on a permanent magnet.

31. A separating chamber of claim 30, wherein each case presents in itself a cylindrical cover with fittings, said cover having a groove into which a sealed disk is inserted, said disk provided with the possibility of rotation around of its own axis by means of a drive.

32. A separating chamber of claim 31, wherein said separating chamber is fixedly secured on a permanent magnet by means of projections and grooves positioned on a magnet ring and on an internal surface of the cases.

33. A separating chamber of claim 32, wherein a magnet is made in the form of a disk half with two built on parts in the form of sectors truncated by 90° half depth with a magnetic field orientation along the central axis of said disk.

34. A separating chamber of claim 31, wherein a disk rotation drive is made in the form of a friction wheel contiguous with the surfaces of two movable disks.

35. A separating chamber of claim 34, wherein the disks are made with the possibility of rotation in counter directions with equal speed.

36. A separating chamber of claim 30, wherein inlet and outlet fittings for separated fluid are located at a median level of the case, and fittings for washing fluid supply are located at the top part of the case.

37. A separating chamber of claim 30, wherein an outlet fitting for a washing fluid is located slightly above a median level of the case.

38. A separating chamber of claim 30, wherein rotating disk in assembly with a cylindrical cover provides a self-contained volume separated by a baffle plate of a falculate shape formed on the interior part of a case cover

39. A separating chamber of claim 38, wherein a baffle plate has a tight contact along its full length with a surface of a rotating disk.

40. A separating chamber of claim 38, wherein an arch-shaped central part of a baffle plate forms a cavity, and a circumferential direct part located radial under a small angle to the median forms an inclined channel.

41. A separating chamber of claim 40, wherein in the bottom of a cavity thus formed there is installed a fitting for exhaust of washing fluid with the oddments of non-reacted components of separated fluid.

42. A separating chamber of claim 40, wherein at the end of an inclined channel there is installed a fitting for exhaust of microcarriers which have reacted.
